(19) Europäisches Patentamt
European Patent Office
Office européen des brevets

(11) EP 2 450 079 B1

(12) **EUROPEAN PATENT SPECIFICATION**

(45) Date of publication and mention
of the grant of the patent:
**25.02.2015 Bulletin 2015/09**

(51) Int Cl.:
*A61M 37/00* (2006.01)    *A61K 9/00* (2006.01)
*A61K 47/36* (2006.01)

(21) Application number: **10794202.1**

(86) International application number:
**PCT/JP2010/061181**

(22) Date of filing: **30.06.2010**

(87) International publication number:
**WO 2011/002034 (06.01.2011 Gazette 2011/01)**

(54) **NEEDLE-LIKE MATERIAL**

NADELARTIGES MATERIAL

MATÉRIAU DE TYPE AIGUILLE

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **01.07.2009 JP 2009156661**

(43) Date of publication of application:
**09.05.2012 Bulletin 2012/19**

(73) Proprietors:
• **Toppan Printing Co., Ltd.
Taito-ku, Tokyo 110-0016 (JP)**
• **Hisamitsu Pharmaceutical Co., Inc.
Tosu-shi, Saga 841-0017 (JP)**

(72) Inventor: **KATO, Hiroyuki
Tokyo 110-0016 (JP)**

(74) Representative: **TBK
Bavariaring 4-6
80336 München (DE)**

(56) References cited:
**EP-A2- 2 005 990        WO-A1-2008/139786
WO-A1-2010/042996        JP-A- 1 313 421
JP-A- 4 356 173          JP-A- 5 321 191
JP-A- 2004 002 320       JP-A- 2008 142 183
US-A1- 2008 114 096**

## Description

Technical Field

The present invention relates to a needle device.

Background Art

The percutaneous absorption method is a method in which the administration of drugs to living bodies such as humans is performed by making these drugs penetrate through the skin into the body. If this method is adopted, drugs can be administered simply to living bodies without giving any pain to the living bodies.

For example, it is proposed in JP-A 48-93192 that a needle device containing many fine projecting portions ($\mu$m order needle) is used for percutaneous administration. When a hole is formed in the skin by using a needle device, for example, the penetration of drugs into living bodies can be promoted. Alternatively, the degree of the penetration can be controlled.

The material constituting the needle device is desired to be one having no adverse influence on human bodies even if a broken needle device is left in the interior of the body. As such a material, biocompatible materials for human body such as chitin and chitosan are proposed, for example, in the gazette of International Publication No. 2008/020632.

A method of producing such a needle device is described, for example, in the gazette of International Publication No. 2008/013282. In this method, first, a precursor plate is obtained by mechanical processing to form a transfer plate from the precursor plate. Using this transfer plate, transfer processing molding is carried out to obtain a needle device.

Alternatively, a needle device can be produced by the method described in the gazette of International Publication No. 2008/004597. In this method, a precursor plate is obtained by the etching method to form a transfer plate from the precursor plate. Using this transfer plate, transfer processing molding is carried out to obtain a needle device.

EP 2 005 990 A2 describes a microneedle device having a coating, wherein the surface of the microneedles and/or the microneedle substrate is partly or entirely coated in fixed state with a coating carrier containing polyvinyl alcohol.

WO 2010/042996 A1, a document according to Art. 54(3) EPC, discloses a method of producing a needle device, wherein tips of projections are immersed in a coating solution which may comprise pectin and alginate.

Disclosure of Invention

The needle device needs to be formed from a material having a low load on living bodies and also needs to be able to form a hole in the skin suitably.

It is an object of the present invention to provide a needle device which is formed from a material having a low load on living bodies and can also form a hole in the skin suitably.

According to one aspect of the present invention, there is provided a needle device including a support portion and a projecting portion extending from the support portion, the projecting portion being constituted of a material containing pectin and an alginate, as defined in claim 1.

Brief Description of Drawings

FIG. 1 is a typical view showing the section of a needle device according to an embodiment of the present invention;
FIG. 2 is a typical view schematically showing an example of a mold;
FIG. 3 is a typical view showing one step in a production method;
FIG. 4 is a typical view showing a step successive to the step shown in FIG. 3;
FIG. 5 is a typical view for explaining a method of measuring the needle strength of a needle device;
FIG. 6 is a typical view for explaining a method of measuring the needle strength of a needle device; and
FIG. 7 is a view showing the correlation between the mixing ratio of materials and strength in a needle device.

Best Mode for Carrying Out the Invention

A needle device in one embodiment will be explained with reference to the drawings.

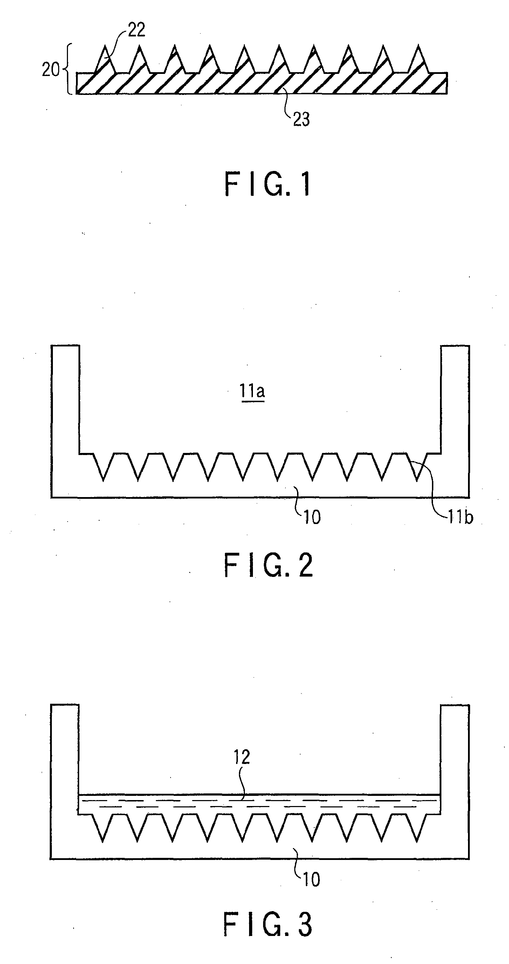

The needle device in one embodiment is a molded article provided with a projecting portion 22 and a support portion 23 that supports the projecting portion as shown in the typical view of FIG. 1. The projecting portion 22 is constituted of a material containing pectin and an alginate.

The support portion 23 preferably has sufficient mechanical strength to support the projecting portion 22 and may have flexibility. When the support portion 23 has flexibility, the needle device can more exactly pierce a flexible subject such as subjects having a curved surface or the skin of a living body. Further, when the support portion 23 has flexibility, the support portion 23 can be molded into a roll-form after the projecting portion 22 is formed.

The support portion 23 may be either a monolayer structure or a multilayer structure. When, for example, the support portion 23 has a multilayer structure including an upper layer and a lower layer and the projecting portion 22 is disposed on the upper layer side, the upper layer is formed of the same material as the projecting portion 22 and the lower layer is formed of a material having higher flexibility than the material constituting the upper layer. Alternately, in the support portion 23, a material having higher malleability than the material constituting the lower layer may be used as the material of the upper layer. Further, as the material of the lower layer, a material which is compressed more easily than the material constituting the upper layer may be used. In these cases, the support portion 23 is easily shaped into a roll form after the projecting portion 22 is formed. When a multilayer structure is adopted, the support portion 23 may further contain an adhesive sheet for fixing layers.

In the support portion 23, the material on the side provided with the projecting portion 22 may be made to be the same as that of the projecting portion 22. In this case, the support portion 23 can be easily formed together with the projecting portion 22 such that it is integrated by molding, which simplifies the production process.

Further, the lowermost layer of the support portion 23 may be a layer having flexibility or bendability. In this case, the projecting portion 22 is scarcely damaged when, for example, layer needle devices 20 are stored in an overlapped state.

The projecting portion 22 may have any form as long as its shape is adequate to pierce subjects such as skin and the form of the projecting portion 22 may be optionally designed. The projecting portion 22 may have, for example, a conical form, pyramid form, columnar form or prism form. Alternatively, the projecting portion 22 may have a pencil form, that is, a form which is a combination of a columnar drum part and a conical top part. There is no limitation to the number of projections 22, and any of a form in which one needle part is disposed on the support portion and a form in which a plurality of needle parts stand together on the support portion is acceptable.

When a plurality of needle parts stands on the support portion, each needle part is preferably arranged array-wise. Here, the term "array-wise" means a situation in which each unit needle part is arranged, and includes patterns such as a lattice arrangement, close packed arrangement, concentric circle arrangement or random arrangement. These forms may be called "Micro-needle arrays".

The length and diameter of the projecting portion 22 are determined corresponding to a subject to be pierced. When the skin of a living body such as a human is pierced, the projection portions 22 are designed to have the diameter range, for example, from several micrometers to hundreds of micrometers and specifically from about 1 $\mu$m to about 300 $\mu$m and a length range from tens of micrometers to hundreds of micrometers and specifically from about 10 $\mu$m to about 1000 $\mu$m. In this case, the aspect ratio of the projecting portion 22 is preferably about 1 to 10.

When, in the case of piercing the skin of a human body, the top of the projecting portion 22 is made to reach the inside of the horny layer without penetrating the projecting portion 22 through the horny layer, the projecting portion 22 is designed to have a length ranging, for example, from about 10 $\mu$m to about 300 $\mu$m and typically from about 30 to 200 $\mu$m.

When, in the case of piercing the skin of a human body, the projecting portion 22 is made to penetrate through the horny layer while preventing the projecting portion 22 from reaching the nervous layer, the projecting portion 22 is designed to have a length ranging, for example, from about 200 $\mu$m to about 700 $\mu$m, typically from about 200 $\mu$m to about 500 $\mu$m, and particularly from about 200 $\mu$m to about 300 $\mu$m.

When, in the case of piercing the skin of a human body, the projecting portion 22 is made to reach the corium, the projecting portion 22 is designed to have a length ranging, for example, from about 200 $\mu$m to about 500 $\mu$m. Further, when, in the case of piercing the skin of a human body, the projecting portion 22 is made to reach the cuticle, the projecting portion 22 is designed to have a length ranging, for example, from about 200 $\mu$m to about 300 $\mu$m.

When the tapered projecting portion 22 decreased in diameter towards its top is made to penetrate through the horny layer of a human body, the maximum angle of the projecting portion 22 is made to be within a range, for example, from 5 to 30 degrees and typically from 10 to 20 degrees.

The needle device 20 may further contain substance to be delivered in the material constituting the projecting portion 22. Alternatively, this needle device 20 may further contain a substance to be delivered carried on the surface of the projecting portion 22. Examples of the substance include pharmacologically active materials and cosmetic compositions. When aromatic substances are used as the substance to be delivered, a fragrance can be imparted in use and the needle device 20 is preferably used as a cosmetic product.

The substance to be delivered can be administered to living bodies even in the case where it is not carried on the projecting portion 22 of the needle device 20. For example, the substance to be delivered is applied to the skin before the piercing using the needle device 20. Or the substance to be delivered is applied to the skin after the piercing using the needle device 20.

Further, the needle device 20 in which the substance to be delivered is carried on the needle part 22 is used to pierce the skin, and then, the substance to be delivered may be further applied to the skin. Alternatively, after the substance to be delivered is applied to the skin, the needle device 20 in which the substance to be delivered is carried on the projecting portion 22 may be used to pierce the skin.

In any case, the substance to be delivered is administered to a subject through a hole formed by pressing the needle device 20 against the skin. Therefore, when pharmacologically active materials are administered by using this needle device 20, besides pharmacologically active materials which are usually administered to living bodies by the percutaneous absorption method, pharmacologically active materials which are supplied by subcutaneous injection or oral administration may also be used as the substance to be delivered. For example, in addition to preparations to be applied to the skin, bio-preparations, typified by vaccines such as influenza virus vaccines, analgesics to be administered to patients with cancers and injections and medicines for oral dosing such as insulin and genetic medicaments may also be used as pharmacologically active materials.

Further, if the needle device 20 is used, medicines which are usually used as injections can be administered without giving any sensation of pain to patients. Moreover, if the needle device 20 is used, medicines which are usually administered orally can be percutaneously administered to patients who have difficulty in swallowing medicines for oral dosing. This means that the needle device 20 is especially suitable for applications for children.

Cosmetic compositions may be used as the substance to be delivered. The cosmetic compositions are compositions to be used as cosmetics or beauty products. Examples of these cosmetic compositions include humectants, colorants, perfumes, or biologically active materials having cosmetic effects (effects of improving wrinkles, pimples, striae gravidarum, and the like, and effects of improving alopecia).

When the needle device 20 is used to supply the substance to be delivered, the depth at which the delivered substance is supplied can be controlled with high accuracy. The retention time of the delivered substance in living bodies varies corresponding to the supply depth.

Because, for example, metabolism is taking place continuously in the horny layer, the substance to be delivered inside the horny layer is excreted from a human body in a relatively short time. The delivered substance on the surface of the skin or in its vicinity can be easily removed by the washing or peeling of the skin. Therefore, for example, in the case where a cosmetic composition is only required to keep its effect for a relatively short time, a relatively small supply depth is proper.

Further, when the projecting portion 22 is made to penetrate through the horny layer to supply a substance to be delivered into the skin, the hole formed in the horny layer is clogged with time. Therefore, the substance to be delivered is scarcely excreted from the living body through the hole, so that the excretion of the delivered substance associated with the metabolism in the horny layer is scarcely caused. The substance to be delivered is removed by the washing or peeling of the skin with difficulty and kept in the living body for a relatively long term. Specifically, in the case where the delivered substance needs to keep its effect for a relatively long period of time, such a relatively large supply depth is proper. Specifically, this corresponds to the case where it is required to maintain the skin coloring effect produced by a cosmetic composition at positions surrounding the peripheries of eyebrows and eyes or the periphery of a lip for a long period of time. Further, this corresponds to the case where skin abnormalities (for example, spots) which are caused at positions deeper than the horny layer are concealed by a cosmetic composition. Moreover, this also corresponds to the case of curing such skin abnormalities by a pharmacologically active material.

In the case where the delivered substance needs to keep its effect for a relatively long period of time, the needle device may be so structured that the projecting portion 22 piercing the skin can be separated from the support portion 23.

In the needle device, at least one of the projecting portion and support portion may be provided with a hollow section. The hollow section may be either a through-hole or non-through-hole. When such a hollow section is provided, the substance to be delivered may be retained inside the needle device. Further, when the needle device is used in applications such as examination of a living body, the organizations (blood, body fluid and the like) of the living body can be well maintained.

This needle device 20 may be used as a micro-needle patch (or a micro-needle device). When the needle device is used in such an application, it is pressed against a living body in such a manner that the projecting portion 22 pierces the skin. At this time, an applicator that fixes the position and direction of insertion of the projecting portion 22 may be used.

In the needle device of this embodiment, as mentioned above, the projecting portion is made from a material containing pectin and an alginate.

When pectin and an alginate are blended, the needle device is particularly improved in mechanical strength in a three-dimensional fine structure of the micrometer-order. In any of the cases using pectin or an alginate singly, it was difficult to produce a needle device having high strength. When pectin and an alginate are blended in use, a needle device having high mechanical strength and biocompatibility can be provided.

Alginic acid is a polysaccharide formed using mannuronic acid and glucaric acid wherein D-mannuronic acid is bonded by $\beta$-1,4 bonding and L-glucaric acid is bonded by $\alpha$-1,4 bonding. This alginate is produced from vegetables such as brown algae by refining and, for example, a part of the alginate is practically used for food additives, showing that it is a material having a low load on living bodies.

The alginate is a compound obtained by ionic bonding between an anion derived from alginic acid and a cation derived from a base. Specific examples of the alginate include sodium alginate, potassium alginate, calcium alginate and ammonium alginate.

Pectin is a complex polysaccharide which contains, as a major component, a polygalacturonic acid obtained by polymerization of galacturonic acid monomers by $\alpha$-1,4- bonding and has a molecular weight of about 50,000 to 360,000. This pectin is produced from vegetables by refining and, for example, a part of pectin is practically used for foods as thickening polysaccharides, showing that it is a material having a low load on living bodies.

This unprecedented improvement in mechanical strength obtained by blending pectin with an alginate was empirically found by the present inventors.

The present inventors inferred that the reason of the improvement in mechanical strength was as follows.

An alginate has an $\alpha$-1,4 bond of D-mannuronic acid and a $\beta$-1,4 bond of L-glucaric acid. For this, molecular chains are easily interweaved with each other and the alginate is therefore put into a state less densified by molecular chains per space. On the other hand, an $\alpha$-1,4 bond of galacturic acid is repeated in pectin. It is considered that this structure of pectin causes pectin to be reduced in the interweaving of molecular chains so that it is put into a state more highly densified by molecular chains per space as compared with alginic acid.

When pectin is blended with an alginate, the molecular chains of pectin are filled in the spaces between the molecular chains of the alginate. It is considered that many molecular chains are interwoven so that the compound mixture is put into a state more highly densified by molecular chains per space. It is inferred that as a result, an unprecedented improvement in the mechanical strength of the mixture material was obtained.

In the needle device used in general skin piercing, the projecting portion has a diameter range from several micrometers to hundreds of micrometers and specifically from about 1 $\mu$m to about 300 $\mu$m and a length range from tens of micrometers to hundreds of micrometers and specifically from about 10 $\mu$m to about 1000 $\mu$m. In this case, the aspect ratio of the projecting portion is about 1 to 10.

When the weight of pectin is "a" and the weight of the alginate is "b", in the case of the needle device having such a projecting portion, the mixing ratio (a/b) is in the following range: $1.00 \leq a/b \leq 2.75$ and preferably in the following range: $1.50 \leq a/b \leq 2.00$.

The needle device of this embodiment may be produced using the following method.

<Step of manufacturing an intaglio>

First, a mold 10 as shown in FIG. 2 is prepared. A first concave portion 11a having a shape corresponding to the support portion 23 of the needle device 20 is formed on one main surface of the mold 10. A second concave portion 11b having a shape corresponding to the projecting portion 22 of the needle device 20 is formed on the bottom surface of the first concave part. The second concave portion 11b may be called a needle pattern.

The mold 10 can be manufactured by various methods.

For example, first, a precursor plate is manufactured by utilizing fine processing technologies. For the fine processing technologies, for example, photolithography, pattern writing using a laser beam or electron beam on a resist layer, wet etching or dry etching using a pattern formed by any of these methods as a mask, sand blast using a pattern having sufficient strength as a mask, laser processing or fine mechanical processing methods such as cutting processing may be used.

In the case where the precursor plate obtained by such a method is a letterpress provided with a needle projecting portion, an intaglio produced from the precursor plate can be utilized as the mold 10. For example, a duplicating plate is produced from the precursor plate made of nickel by electrodeposition and may be utilized as the mold 10. Alternatively, in the case where the precursor plate has sufficient heat resistance, a molten thermoplastic resin is supplied to the precursor plate and in this condition, the thermoplastic resin is cooled to obtain a duplicating plate, which may be used as the mold 10.

Further, in the case where the obtained precursor plate is an intaglio provided with a concave portion corresponding to the needle projecting portion, the precursor plate itself can be utilized as the mold 10. Further, a duplicating plate is produced from the precursor plate and this duplicating plate may be utilized as the mold 10.

<Step of preparing a needle device material>

An aqueous solution containing pectin and an alginate is used as a needle device material (hereinafter referred to as "coating solution"). An unprecedented improvement in the mechanical strength is obtained in a fine three-dimensional structure of the micrometer-order which is obtained using this aqueous solution. This fact was discovered by the present inventors.

Pectin and an alginate are soluble in water and they are prepared in the form of a solution. Here, the liquid state means that the solution may have fluidity to such an extent as to be able to flow into the intaglio and may be made into a gel form.

The total concentration of pectin and alginate in the coating solution may be arbitrarily designed. The total concentration of pectin and alginate in the coating solution is preferably designed to be in a range from 1 to 10 wt% to avoid handling difficulty caused by a rise in the viscosity of the coating solution. If pectin and alginate are contained in such a concentration, a desired needle device can be formed.

When the weight of pectin is "a" and the weight of the alginate is "b" in the coating solution, the mixing ratio (a/b) is in the following range: $1.00 \leq a/b \leq 2.75$ and preferably in the following range: $1.50 \leq a/b \leq 2.00$.

Pharmacologically active substances, cosmetic compositions and the like may be compounded in the aqueous solution containing pectin and alginate as substances to be delivered.

<Step of filling the coating solution>

As shown in FIG. 3, the coating solution is filled in the concave portion 11b and a part of concave portion 11a in the mold 10 to form a layer 12 made from the coating solution. When the coating solution is filled, a known method may be selected appropriately corresponding to the shape and dimension of the mold 10. Examples of the method include a spin coating method, a method using a dispenser, and a casting method. The coating solution may be filled in the mold 10 in a reduced pressure atmosphere or under vacuum.

<Drying and solidifying step>

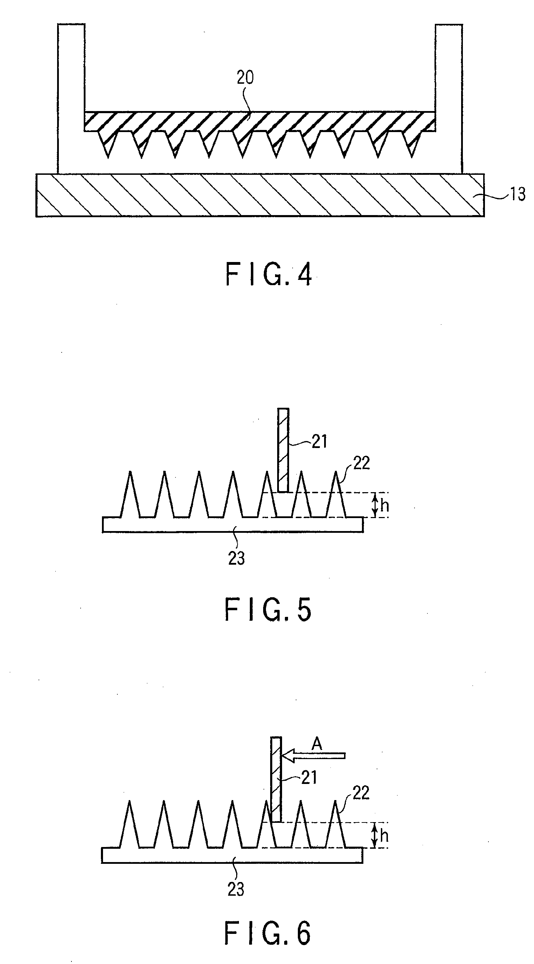

The layer 12 of the coating solution filled in the mold 10 is dried into a solid by a heat source 13 to obtain a film-like needle device 20 as shown in FIG. 4. As the drying method, natural drying, bottom heating using a hot plate, drying using hot air drying, or the like may be optionally selected corresponding to the environment.

The heating temperature and time may be properly determined corresponding to, for example, the composition of the coating solution, and the characteristics of the pharmacologically active substance and cosmetic composition.

After heating for a prescribed time, the intaglio is removed to obtain a needle device of this embodiment. The intaglio can be removed by physical force. Alternatively, a chemical treatment may be performed to selectively dissolve the intaglio.

Though the case where the needle device 20 is used in medical applications or in cosmetic or beauty applications is described here, the needle device 20 may be used in other applications. Further, though the case where the needle device 20 is utilized as a piercing tool for piercing a subject is described here, the needle device 20 may be used for other purposes. The needle device 20 may be utilized in, for example, microelectromechanical system (MEMS) devices, optical members, sample fixtures and drug discovery.

Though the needle device of one embodiment will be explained in detail below, the present invention is not limited to the embodiment.

First, fine mechanic processing of a silicon substrate was carried out to form a dent-projection structure in which 36 regular square pyramids were arranged in the form of a 6 x 6 perfect grid, thereby obtaining a precursor plate having a needle projecting portion. Each regular square pyramid was formed such that each side of its bottom surface had a length of 60 $\mu$m and a height of 150 $\mu$m. The distance between the regular square pyramids neighbored in the direction of the line and the distance between the regular square pyramids neighbored in the direction of the row were each designed to be 1 mm.

A nickel film 500 $\mu$m in thickness was formed on the dent-projection structure of the obtained precursor plate which was the intaglio provided with the needle projecting portion by the plating method. Then, an aqueous potassium hydroxide containing 30% by mass of potassium hydroxide was heated to 90°C and this aqueous solution was used as an etching solution to perform wet etching, thereby removing the precursor plate from the nickel layer. In this manner, a mold was obtained which was made of nickel and was provided with a needle pattern corresponding to the above 36 projecting portions each having a regular square pyramid from on one main surface thereof.

As the coating solution, an aqueous solution containing pectin and sodium alginate was prepared. The total concentration of pectin and sodium alginate in the aqueous solution was designed to be 7.5% by weight. The weight "a" of pectin and the weight "b" of sodium alginate were varied to prepare 6 types of aqueous solutions different in mixing ratio (a/b) as the total concentration. The mixing ratios (a/b) were 0.25, 0.50, 1.00, 2.00, 4.00 and 9.00.

The obtained coating solution was filled in the aforementioned mold by the spin coating method as shown in FIG. 3. This mold was mounted on a hot plate as the heat source 13 as shown in FIG. 4. The coating solution was heated at 120°C for 10 minutes to dry the coating film into a solid.

After the coating film was dried into a solid, the mold 10 was removed to obtain 6 needle devices of Examples. In the needle devices obtained in the examples, the mixing ratios of pectin to sodium alginate (a/b) were 0.25, 0.50, 1.00, 2.00, 4.00 and 9.00.

<Reference Example 1>

A needle device was produced in the same manner as above except that an aqueous sodium alginate was used as the coating solution. The concentration of sodium alginate in the aqueous solution was 7.5% by weight.

<Reference Example 2>

A needle device was produced in the same manner as above except that an aqueous pectin solution was used as the coating solution. The concentration of pectin in the aqueous solution was 7.5% by weight.

<Test for measuring strength>

The strength of the obtained needle device was measured using a needle device strength tester.

The needle strength was measured by using a needle device strength measuring meter. The needle device strength measuring meter is the following measuring device having a measuring needle sensitive to load. The measuring needle is made to be in contact with a detecting position at a predetermined measuring height to destroy the needle part to measure the maximum load to be applied to the measuring needle at this time.

Specifically, as shown in FIG. 5, the measuring needle 21 is disposed between the needle parts 22 on the support portion 23 while keeping the measuring needle 21 at the height h. Here, the measuring height h was

set to 10 μm. Then, as shown in FIG. 6, the measuring needle is made to be in contact with the needle part 22 while keeping the measuring height h to apply a load in a horizontal direction as shown by the arrow A. The maximum load measured when the needle part 22 was destroyed and lay sideways was defined as the needle strength.

The obtained results are described together with the mixing ratio (a/b) of the coating solution in the following Table 1. [Table 1]

Table 1

| Mixing ratio a/b | Average strength (g) |
|---|---|
| Reference Example 9.00 | 44.4 |
| Reference Example 4.00 | 49.1 |
| 2.00 | 71.6 |
| 1.00 | 60.0 |
| Reference Example 0.50 | 37.4 |
| Reference Example 0.25 | 34.2 |
| Reference Example 1 | 27.6 |
| Reference Example 2 | 9.6 |

As shown in the above Table 1, the needle device produced using a material containing pectin and sodium alginate has a higher strength than the needle device (Reference Example 1) produced using single sodium alginate and the needle device (Reference Example 2) produced using single pectin.

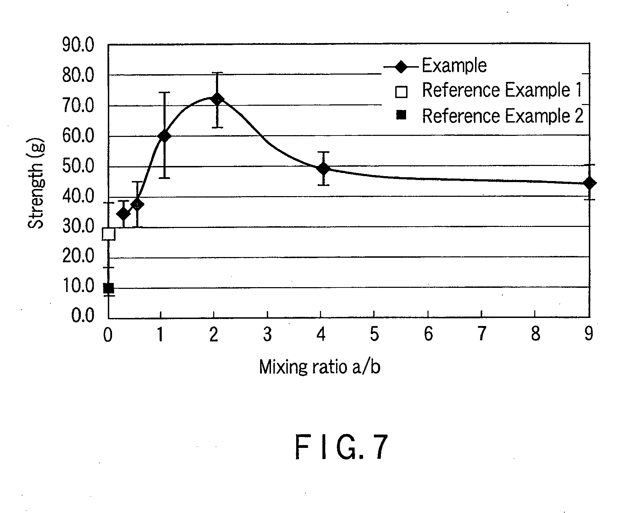

FIG. 7 is a graph for explaining the strength of the needle device of the example wherein the strength is plotted as a function of the mixing ratio (a/b) with the abscissa representing the mixing ratio (a/b), and the ordinate representing the strength. For reference, the strengths obtained in Reference Examples 1 and 2 are shown on the ordinate in FIG. 7.

It is found from FIG. 7 that the mixing ratio (a/b) at which the strength is raised exists. When the mixing ratio is close to 1.75 ($1.50 \leq a/b \leq 2.00$), the needle device has a particularly large strength.

It was confirmed that a needle device was obtained which was sharply improved when the mixing ratio was in the following range: $1.00 \leq a/b \leq 2.75$ and exhibited higher strength when the mixing ratio was in the following range: $1.50 \leq a/b \leq 2.00$.

Industrial Applicability

The needle device of this embodiment can be exploited in various fields which need fine needle devices. It is expected to be applied as needle devices used in applications, such as microelectromechanical system (MEMS) devices, optical members, sample fixtures, drug discovery, medicines, cosmetic products and beauty products.

Reference Signs List

10: Mold; 11a: First concave portion;
11b: Second concave portion;
12: Layer made of a coating solution;
13: Heat source; 20: Needle device;
21: Measuring needle; 22: Projecting portion;
23: Support portion; h: Measuring height

**Claims**

1. A needle device comprising:

a support portion and a projecting portion extending from the support portion, the projecting portion being constituted of a material comprising pectin and an alginate, wherein in the material, a weight "a" of the pectin and a weight "b" of the alginate satisfy the following relation:

$$1.00 \leq a/b \leq 2.75.$$

2. The needle device according to claim 1, wherein, among the support portion, a material of a main surface on which the projecting portion is disposed is the same as the material of the projecting portion.

3. The needle device according to claim 1 or 2, wherein a height of the projecting portion is in a range from 10 to 1000 μm.

4. The needle device according to Claim 1, wherein in the material, the weight "a" of the pectin and the weight "b" of the alginate satisfy the following relation:

$$1.50 \leq a/b \leq 2.00.$$

5. The needle device according to any one of claims 1 to 4, wherein the alginate is sodium alginate.

6. The needle device according to any one of claims 1 to 5, further comprising a substance to be delivered, the substance being supported by the projecting portion.

7. The needle device according to any one of claims 1 to 6, wherein the material further comprises a substance to be delivered.

**8.** The needle device according to claim 6 or 7, wherein the substance is a pharmacologically active substance.

**9.** The needle device according to claim 6 or 7, wherein the substance is a cosmetic composition.

**Patentansprüche**

**1.** Nadelvorrichtung, umfassend:

einen Trägerteil und einen hervorstehenden Teil, der sich aus dem Trägerteil erstreckt, wobei bei der hervorstehende Teil aus einem Material aufgebaut ist, das Pectin und ein Alginat umfasst, wobei in dem Material ein Gewicht "a" des Pectins und ein Gewicht "b" des Alginats die folgende Beziehung erfüllt:

$$1,00 \leq a/b \leq 2,75.$$

**2.** Nadelvorrichtung nach Anspruch 1, wobei, unter dem Trägerteil, ein Material einer Hauptoberfläche, auf welcher der hervorstehende Teil angeordnet ist, das gleiche wie das Material des hervorstehenden Teils ist.

**3.** Nadelvorrichtung nach einem der Ansprüche 1 oder 2, wobei eine Höhe des hervorstehenden Teils in einem Bereich von 10 bis 1000 $\mu$m ist.

**4.** Nadelvorrichtung nach Anspruch 1, wobei in dem Material das Gewicht "a" des Pectins und das Gewicht "b" des Alginats die folgende Beziehung erfüllen:

$$1,50 \leq a/b \leq 2,00.$$

**5.** Nadelvorrichtung nach einem der Ansprüche 1 bis 4, wobei das Alginat Natriumalginat ist.

**6.** Nadelvorrichtung nach einem der Ansprüche 1 bis 5, die ferner eine abzugebende Substanz umfasst, wobei die Substanz durch den hervorstehenden Teil getragen wird.

**7.** Nadelvorrichtung nach einem der Ansprüche 1 bis 6, wobei das Material ferner eine abzugebende Substanz umfasst.

**8.** Nadelvorrichtung nach Anspruch 6 oder 7, wobei die Substanz eine pharmakologisch aktive Substanz ist.

**9.** Nadelvorrichtung nach Anspruch 6 oder 7, wobei die Substanz eine kosmetische Zusammensetzung ist.

**Revendications**

**1.** Dispositif d'aiguille comprenant :

une portion de support et une portion faisant saillie s'étendant à partir de la portion de support, la portion faisant saillie étant constituée d'un matériau comprenant de la pectine et un alginate, dans lequel dans le matériau, un poids "a" de la pectine et un poids "b" de l'alginate satisfont la relation suivante :

$$1,00 \quad a/b \quad 2,75.$$

**2.** Dispositif d'aiguille selon la revendication 1, dans lequel, parmi la portion de support, un matériau d'une surface principale sur laquelle la portion faisant saillie est disposée est identique au matériau de la portion faisant saillie.

**3.** Dispositif d'aiguille selon la revendication 1 ou 2, dans lequel une hauteur de la portion faisant saillie se trouve dans un intervalle de 10 à 1 000 $\mu$m.

**4.** Dispositif d'aiguille selon la revendication 1, dans lequel dans le matériau, le poids "a" de la pectine et le poids "b" de l'alginate satisfont la relation suivante :

$$1,50 \quad a/b \quad 2,00.$$

**5.** Dispositif d'aiguille selon l'une quelconque des revendications 1 à 4, dans lequel l'alginate est l'alginate de sodium.

**6.** Dispositif d'aiguille selon l'une quelconque des revendications 1 à 5, comprenant de plus une substance à administrer, la substance étant supportée par la portion faisant saillie.

**7.** Dispositif d'aiguille selon l'une quelconque des revendications 1 à 6, dans lequel le matériau comprend de plus une substance à administrer.

**8.** Dispositif d'aiguille selon la revendication 6 ou 7, dans lequel la substance est une substance pharmacologiquement active.

**9.** Dispositif d'aiguille selon la revendication 6 ou 7, dans lequel la substance est une composition cosmétique.

FIG. 1

FIG. 2

FIG. 3

F I G. 4

F I G. 5

F I G. 6

F I G. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 48093192 A
- WO 2008020632 A
- WO 2008013282 A

- WO 2008004597 A
- EP 2005990 A2
- WO 2010042996 A1